# EUROPEAN PATENT SPECIFICATION

(11) **EP 1 731 160 B1**
(45) Date of publication and mention of the grant of the patent: **11.07.2012**
(21) Application number: 05719531.5
(22) Date of filing: 25.02.2005
(51) Int. Cl.: A61K 36/00

(54) **CANCER METASTASIS INHIBITORY COMPOSITION**
ZUSAMMENSETZUNG ZUR HEMMUNG VON KREBSMETASTASEN
COMPOSITION INHIBITOIRE POUR METASTASE CANCERIGENE

(30) Priority: 27.02.2004 JP 2004054554
(43) Date of publication of application: 13.12.2006
(73) Proprietor: Fuji Oil Company, Ltd., Osaka-shi, Osaka 542-0086 (JP); Kobayashi, Hiroshi, Kashihara-shi, Nara 634-0005 (JP)
(72) Inventor: KOBAYASHI, Hiroshi, Kashihara-shi Nara 634-0005 (JP); YOSHIDA, Ryuji, c/o Fuji Oil Company, Limited, Osaka 598-8540 (JP); FUKUDA, Yoichi, c/o Fuji Oil Company, Limited, Han, Osaka 598-8540 (JP)
(74) Representative: Hayes, Adrian Chetwynd
(86) International application number: PCT/JP2005/003130
(87) International publication number: WO 2005/082389

(56) References cited:
- EP-A- 0 301 122
- WO-A1-97/25422
- JP-A- 7 010 772
- JP-A- 7 010 773
- JP-A- 9 100 298
- JP-A- 10 066 572
- JP-A- 62 294 622
- JP-A- 2000 086 531
- KOBAYASHI HIROSHI ET AL: "A Kunitz-type protease inhibitor, bikunin, inhibits ovarian cancer cell invasion by blocking the calcium-dependent transforming growth factor-beta1 signaling cascade." JOURNAL OF BIOLOGICAL CHEMISTRY, vol. 278, no. 10, 7 March 2003 (2003-03-07), pages 7790-7799, XP002538819 ISSN: 0021-9258
- MESSINA M ET AL: "THE ROLE OF SOY PRODUCTS IN REDUCING RISK OF CANCER" JOURNAL OF THE NATIONAL CANCER INSTITUTE, OXFORD UNIVERSITY PRESS, GB, vol. 83, no. 8, 17 April 1991 (1991-04-17), pages 541-546, XP002056624 ISSN: 0027-8874
- KOBAYASHI HIROSHI ET AL: "A soybean Kunitz trypsin inhibitor suppresses ovarian cancer cell invasion by blocking urokinase upregulation" CLINICAL & EXPERIMENTAL METASTASIS, vol. 21, no. 2, 2004, pages 159-166, XP002538820 ISSN: 0262-0898
- WU J. ET AL: 'Enhanced, Vascular Permeability in Solid Tumor Involving Peroxynitrite and Matrix Metalloproteinases.' JPN. J. CANCER RES. vol. 92, no. 4, 2001, pages 439 - 451, XP002989591

## Description

### Technical Field

The present invention relates to a composition for use in inhibiting cancer metastasis, not a composition having an anti-cancer effect.

### Background Art

In Japan, cancer has consistently ranked first among causes of death since 1981, and accounts for one fourth of the total causes of death. In the present situation that cancer is the number-one cause of death, eradication of cancer is the greatest challenge for mankind. At present, the total recovery rate for cancer is 50%. In the case of solid tumors, the recovery rate for advanced cancer is only 10% even today. The reason why cancer patients die is that cancer metastasizes to solid organs. Therefore, development of effective cancer metastasis inhibitors will result in great improvement of the prognoses of patients with cancer, especially, advanced cancer. It is ideal that effective cancer metastasis inhibitors have no toxicity and can be provided at low cost because continuing administration of a drug for a long time is required to inhibit cancer metastasis.

One of the inventors of the present invention, in an earlier study, found bikunin, a physiological substance which specifically inhibits cancer metastasis, in human amniotic fluid and reported that animal tests with bikunin showed a good survival rate without side effect (Kobayashi H., Shinohara H., Gotoh J., Fujie M., Fujishiro S., Terao T.: Anti-metastatic therapy by urinary trypsin inhibitor in combination with an anti-cancer agent. Br. J. Cancer 72:1131-1137, 1995). However, in order to administrate bikunin to a cancer patient for a long time, a large amount of human amniotic fluid must be collected to purify bikunin therefrom. This is very expensive and impractical, and imposes a heavy financial burden on a cancer patient.

Trypsin inhibitors are contained in soybeans, and especially in soybean whey, which is a byproduct of isolated soybean protein preparation, in relatively abundance. Soybean trypsin inhibitors are classified into Kunitz trypsin inhibitors (referred to as "KTI") and Bowman-Birk trypsin inhibitors (referred to as "BBI") (Tokuji Ikenaka, "Structure and inhibitory mechanism of leguminous plant protease inhibitors", Tanpakushitsu, Kakusan, Koso (Protein, Nucleic Acid and Enzyme), Vol.27, No.12, 1738-1746, 1982). JP-A 6-145061 reports that a fraction of soybean whey has an anti-cutaneous cancer effect. JP-A 7-10773 reports that KTI has an effect of enhancing carcinostatic activity.

However, both of the reports disclose only that a soybean trypsin inhibitor has the effect of a carcinostatic agent that inhibits the development of cancer itself and a soybean trypsin inhibitor in combination with another carcinostatic agent has an effect of enhancing carcinostatic activity. Such carcinostatic activity only shows an effect of directly inhibiting the proliferation of cancer cells and an effect of directly killing cancer cells. There is no disclosure that a soybean trypsin inhibitor inhibits metastasis and recurrence of cancer resulting from the growth of an already formed small metastatic lesion.

EP 0,301,122 A1 relates to an agent for inhibiting retention of cancerous ascites/hydrothorax, inhibiting exaggeration of inflammatory edema, and enhancing activities of a carcinostatic substance by using a soybean Kunitz trypsin inhibitor. The effect of this agent is the inhibition of cancer metastasis. In particular,
EP 0,301,122 A1 investigates survival rates by intraperitoneal inoculation of Sarcoma 180 cells to mice on day 0, followed by intraperitoneal administration of Kunitz trypsin inhibitor or Bowman-Birk trypsin inhibitor (both are of soybean origin) with varying timings. Inhibition of ascetic retention is confirmed only for the Kunitz trypsin inhibitor administering group.

Kobayashi H et al., Journal of Biological Chemistry, vol. 278, no. 10, 7 March 2003, pages 7790-7799 discloses that a Kunitz-type protease inhibitor, Bikunin, inhibits ovarian cancer cell invasion by blocking the calcium-dependent transforming growth factor-betal signaling cascade.

Messina M et al, Journal of the National Cancer Institute, Oxford University Press, GB, vol. 83, no. 8, 17 April 1991, pages 541-546 describes that soybean-derived Bowman-Birk protease inhibitor either inhibits or prevents development of experimentally induced colon, oral, lung, liver and esophageal cancers.

### Disclosure of Invention

### Problem to be solved by the invention:

As described above, a cancer metastasis inhibitor that exhibits a potent cancer metastasis inhibiting effect comparable to bikunin and can be obtained in a large amount at a low cost has been never known. That is, an objective of the present invention is to provide such a cancer metastasis inhibitor.

### Means for solving the problem:

The inventors of the present invention, in view of the above-mentioned problems, performed a homology search for the molecular structure of bikunin, and confirmed that the structure of bikunin has extremely high homology with the structure of Kunitz trypsin inhibitors (hereinafter, referred to as "KTI") from soybean. Then, they examined the cancer metastasis inhibitory effect of KTI by administration thereof, and found that KTI has a potent cancer metastasis inhibitory activity. KTI is present in a large amount in soybean whey, which is yielded in a large amount in the production of isolated soybean protein and the like from soybean, and soybean whey can be obtained at a low cost. Therefore, if a large amount of KTI is extracted and purified from soybean whey, KTI is clinically applicable and also applicable to health food products.

That is, the present invention provides:
(1) A composition comprising a soybean Kunitz trypsin inhibitor as an active ingredient for use in inhibiting cancer metastasis;
(2) The composition according to the above (1), which comprises soybean whey as a source of the soybean Kunitz trypsin inhibitor;
(3) The composition according to the above (1), which comprises a trypsin inhibitor concentrate obtained from soybean whey as a source of the soybean Kunitz trypsin inhibitor;
(4) The composition according to the above (3), wherein a Bowman-Birk trypsin inhibitor is separated and removed as much as possible from the trypsin inhibitor concentrate obtained from soybean whey;
(5) The composition according to the above (1), which is a food product or medicine;

The composition according to the above (1) further comprising an anti-cancer ingredient and/or a cancer preventive ingredient.

### Effects of the Invention

According to the present invention, a composition that exhibits a potent cancer metastasis inhibitory effect, has no side effect and is clinically applicable can be provided in a large amount at a low cost by purifying a soybean KTI from soybean whey, which has never been sufficiently utilized. Therefore, the composition of the present invention is extremely useful in the case of relatively early cancer wherein the possibility of metastasis can not be eliminated, and for inhibiting cancer from further metastasizing in cancer patients in whom a metastatic lesion has already formed, and can be administrated as a medicine or functional food product for a long time.

### Best Mode for Carrying Out the Invention

### (Preparation method of KTI)

KTI, which is an active ingredient of the composition of the present invention, is mainly contained in the whey component of soybeans. Therefore, the composition of the present invention preferably comprises soybean whey as a source of KTI. More preferred source of KTI is a trypsin inhibitor concentrate obtained from soybean whey. More preferably, BBI is separated and removed as much as possible from the trypsin inhibitor concentrate. The above-described source may be prepared by a known method and, for example, is preferably prepared by, but not limited to, the following method.

The soybean whey to be used includes soybean whey containing a soybean trypsin inhibitor. In other words, soybean whey that does not undergo such heat history as deactivates a soybean trypsin inhibitor during a process of producing said soybean whey is preferably used.

Soybean whey is a byproduct in a process of producing a isolated soybean protein or a concentrated soybean protein from soybean, and is also referred to as soybean molasses. Soybean whey undergoing less heat history can be obtained as a solution, for example, by extracting low-denatured defatted soybeans with water at around neutral pH, removing the okara component (soybean curd refuse) to obtain defatted soymilk, allowing soybean proteins to aggregate and precipitate at around the isoelectric point (e.g., around pH 4.5), and then removing them from the defatted soymilk.

Soybean whey obtained as a byproduct in an industrial process of producing a isolated soybean protein can be advantageously used as a large amount of raw material with a stable quality. Typically, such soybean whey has a solid content of about 3%. The composition of the solid content (hereinafter, also referred to as "dry%") consists of about 20% of crude protein, about 20% of ash and about 60% of carbohydrate. Even when the extraction rate with water from defatted soybeans is varied, the composition of the solid content in soybean whey tends not to vary. And, if the solid content in soybean whey is around 3%, then a soybean trypsin inhibitor is contained at approximately 5 units/ml of its activity. Accordingly, suitable soybean whey that can be used in the present invention has at least 3 units/ml or more of the activity. In the present invention, the soybean trypsin inhibitor activity is quantified by the BAPA method based on the official method of A.O.C.S.

Next, a soybean trypsin inhibitor concentrate can be obtained by separating trypsin inhibitors from soybean whey. For the separation, various methods can be used, but a method comprising precipitating them by salting-out and then collecting the precipitates is convenient. For example, when soybean whey comprises 3% of a solid content, trypsin inhibitors can be collected as aggregated precipitates by adding sodium sulfate to the soybean whey so that the concentration of sodium sulfate is 14%, and keeping the pH of the soybean whey at a range of pH 1.7 to 5.2, preferably a range of pH 2.7 to 4.8.

Alternatively, a method described in JP-A 2004-313170 can be used. In this method, trypsin inhibitors can be collected as aggregated precipitates by concentrating soybean whey at such temperature that the trypsin inhibitors are not deactivated, instead of adding a salt, to increase the percentage of a solid content to around 30 to 50% by weight, and keeping the pH of the soybean whey under a acidic condition, preferably at a range of pH 1.7 to 5.2, more preferably at a range of pH 2.7 to 4.8 to salt out the trypsin inhibitors as inner salts. The method has the advantage that there is no need to desalt. The concentration can be accomplished by a known method. Concentrating under a reduced pressure prevents an increase in temperature, so that the concentration can be efficiently accomplished.

Further, alternatively, a method described in Japanese Patent Application No. 2004-22933 can be used. In this method, the above-described salting out and the above-described concentrating are combined, and trypsin inhibitors are collected by concentrating soybean whey to 10 to 30% by weight of a solid content, adding 3% by weight or more of a salt to the concentrated soybean whey, and allowing trypsin inhibitors to precipitate as aggregated precipitates under an acidic condition.

Since the soybean trypsin inhibitor concentrate thus obtained is also concentrated in terms of KTI, it can be used in the present invention as a KTI concentrate.

Further, in order to increase the potency of KTI in the soybean trypsin inhibitor concentrate, it is preferable to remove BBI as much as possible from the trypsin inhibitor concentrate and thereby make the concentration of KTI higher. The separation of KTI from BBI can be accomplished by a known method. In the separation, it should be noted that although a Kunitz trypsin inhibitor (KTI) and a Bowman-Birk trypsin inhibitor (BBI) have isoelectric points of pH 4.5 and pH 4.2 respectively, adjustments of the pH of soybean whey to respective isoelectric points for trying the isoelectric point precipitation result in no or less aggregated precipitates of the desired substance. Therefore, the following separation method described in JP-A 2004-313170 is preferable.

The aggregated precipitates of trypsin inhibitors can be further fractionated into a solid fraction and a liquid fraction by adding water to the aggregated precipitates and partially dissolving them at a range of pH 2.0 to 4.8, preferably at a range of pH 3.0 to pH 4.4. The liquid fraction obtained by the solid-liquid fractionation can be a BBI concentrate, and the solid fraction can be a KTI concentrate. The reason that BBI and KTI can be separated into a supernatant and a precipitate by this fractionation method is that the salt concentration becomes lower than that of the concentrated whey by an addition of water, so that BBI can redissolve while KTI can not dissolve. The amount of water added is 1.5 or more times, preferably 2 to 10 times, most preferably 2.5 to 4 times the amount of the aggregated precipitates (containing water). The KTI thus obtained may be used as it is, or may be used in the wet or dried form after it is redissolved at neutral pH.

The composition of the present invention comprises the above-described KTI as an active ingredient. The composition of the present invention is a food product or medicine. When the composition of the present invention is administered as a medicine, the above-described KTI is administered alone or in various dosage forms prepared by mixing it with a pharmaceutically acceptable carrier. In the case of any dosage form, the KTI is formulated together with a pharmaceutically acceptable carrier according to a usual method to prepare a cancer metastasis inhibiting agent. Examples of a carrier used include those commonly used in usual agents, for example, diluents and excipients, such as for example, fillers, extenders, binders, disintegrants, surfactants and lubricants.

When the cancer metastasis inhibiting agent of the present invention is used for the purpose of cancer metastasis inhibition in a mammal including human, the dosage unit form thereof is not specifically limited and can be appropriately selected depending on a therapeutic purpose. Specific examples of the dosage unit form include for example, tablet, pill, powder, solution, suspension, emulsion, granule, capsule, suppository, injection, ointment and patch.

When the cancer metastasis inhibiting agent of the present invention is formulated into a tablet form, examples of a carrier to be used include excipients such as for example, lactose, saccharose, sodium chloride, glucose, urea, starch, calcium carbonate, kaolin, crystalline cellulose and silicic acid; binders such as, for example, water, ethanol, propanol, simple syrup, glucose solution, starch solution, gelatin solution, carboxymethylcellulose, shellac, methylcellulose, potassium phosphate and polyvinylpyrrolidone; disintegrants such as for example, dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch and lactose; degradation inhibitors such as saccharose, stearic acid, cacao butter, hydrogenated oil; absorption enhancers such as, for example, quaternary ammonium base and sodium lauryl sulfate; moisturizing agents such as, for example, glycerin and starch; absorbents such as , for example, starch, lactose, kaolin, bentonite and colloidal silicic acid and; lubricants such as, for example, purified talc, stearate, boric acid powder and polyethylene glycol.

Further, a tablet may be a coated tablet with a common coating, for example, a sugarcoated tablet, a gelatin-encapsulated tablet, an enteric-coated tablet, a film-coated tablet and a multilayered tablet, as needed.

When the cancer metastasis inhibiting agent of the present invention is formulated into a pill form, examples of a carrier to be used include excipients such as, for example, glucose, lactose, starch, cacao butter, hardened vegetable oil, kaolin and talc; binders such as, for example, powdered gum arabic, tragacanth powder, gelatin and ethanol; and disintegrants such as, for example, laminaran and agar.

A capsule is prepared by mixing the active ingredient of the present invention with various carriers described above and filling the mixture into, for example, a hard gelatin capsule or a soft capsule.

When the cancer metastasis inhibiting agent of the present invention is formulated into a suppository form, examples of a carrier to be used include, for example, polyethylene glycol, cacao butter, hard butter, esters of higher alcohols, gelatin and semisynthetic glyceride, such as glyceride obtained by transesterification.

When the cancer metastasis inhibiting agent of the present invention is prepared as an injection, a solution, an emulsion and a suspension are preferably sterilized and isotonic to blood. In preparation of these dosage forms, examples of a diluent to be used include, for example, water, aqueous lactic acid solution, ethyl alcohol, propylene glycol, ethoxylated isostearyl alcohol, polyoxylated isostearyl alcohol and polyoxyethylene sorbitan fatty acid esters. In this case, the pharmaceutical preparation may contain sodium chloride, glucose or glycerin in a sufficient amount to prepare an isotonic solution. For example, a conventional solubilizing agent, buffer and soothing agent may be also added to the pharmaceutical preparation.

When the cancer metastasis inhibiting agent of the present invention is formulated into an ointment form such as paste, cream or gel, examples of a diluent to be used include white petrolatum, paraffin, glycerin, cellulose derivatives, polyethylene glycol, silicon and bentonite.

The above-described preparations may further contain, for example a colorant, a preservative, a perfume, a flavor, a sweetener, and other drugs, as needed.

The amount of the KTI contained in the cancer metastasis inhibiting agent of the present invention is not specifically limited, and may be appropriately selected depending on the daily dose. It is usually 1 to 90% by weight of the preparation.

An administration method of the cancer metastasis inhibiting agent of the present invention is not specifically limited, and is determined depending, on ,for example, a dosage form, the age, sex and other conditions of a patient, and the severity of a patient's symptom. For example, tablets, pills, powders, solutions, suspensions, emulsions, granules and capsules are orally administrated. Suppositories are intrarectally administrated. Injections are intravenously administered alone or in combination with a conventional replacement fluid such as, for example, glucose or amino acid, or as needed, administered intraarterially, intramuscularly, intradermally, subcutaneously or intraperitoneally alone. Ointments are applied to, for example, skin and intraoral mucosa.

The dose of the active ingredient contained in the cancer metastasis inhibitor of the present invention is not specifically limited, and can be appropriately selected depending on, for example, usage, the age, sex and other conditions of a patient, the severity of a disease, and a purpose. As a guide, the dose of the active ingredient may be usually 0.2 to 30 g/day of KTI, but the dose may be out of the range between the upper limit and the lower limit. The cancer metastasis inhibiting agent of the present invention can be administered once a day or in 2 to 4 divided doses per day.

The type of cancer to which the cancer metastasis inhibiting agent of the present invention is applicable is not specifically limited. For example, the cancer metastasis inhibiting agent of the present invention can be used as a metastasis inhibitor of a malignant tumor such as , for example, lung cancer, ovarian cancer, malignant melanoma, uterus cancer, small and large intestinal cancer, breast cancer, fibrosarcoma or leukemia.

The above described KTI has a potent cancer metastasis inhibitory effect and is an ingredient obtained from soybeans that are a traditional food. Therefore, the KTI can be added into various food products as an active ingredient to prepare food products for use in inhibiting cancer metastasis. The amount of the KTI added to a food product may be such an amount that the amount equal to the dose of KTI described above as a guide can be ingested from the food product, or in the case of relatively early cancer, it may be such an amount that an amount less than the dose of KTI described above as a guide can be ingested from the food product for prophylactic purposes. The food products to which the KTI is applicable are not specifically limited, and include various food products including, for example, oil-in-water emulsified food products such as cream; water-in-oil emulsified food products such as margarine; edible oils; beverages such as tea beverages, refreshing beverages and milk beverages; dairy products such as milk, cheese and yogurt; soybean products such as soy milk, fermented soy milk, soybean protein drinks, tofu, natto (fermented soybeans), aburage (deep-fried tofu), atsuage (deep-fried pressed tofu) and ganmodoki (fried tofu ball containing bits of various kinds of vegetables); processed meats such as hamburger, meatball, fried chicken and nugget; various delicatessen foods; confectionery such as baked goods, chocolate, cake, frozen dessert, cereal, candy, gum and tablet; breads such as bread, sweet bread and doughnut; and rice dishes such as cooked rice, sushi and mochi (rice cake).

The composition of the present invention can be used in combination with other active ingredients having cancer metastasis inhibitory effects such as bikunin, if desired.

The composition of the present invention can further comprise an anti-cancer ingredient and/or cancer preventive ingredient, and thereby can become a composition for anti-caner and/or prevention of cancer and inhibition of cancer metastasis which inhibits totally from the development of cancer to the growth and metastasis of cancer.

Further, the composition of the present invention can be combined with a composition comprising an anti-cancer ingredient and/or cancer preventive ingredient, and thereby a combined composition for anti-caner and/or prevention of cancer and inhibition of cancer metastasis can be obtained.

Examples of the anti-cancer ingredient and/or cancer preventive ingredient have no specific limitation. When the composition is a food product, one or more of ingredients approved for addition to food, for example, one or more selected from oligosaccharides such as soybean oligosaccharide and manno-oligosaccharide, useful microorganisms such as bifidobacteria and lactobacillus, polyphenols such as isoflavone, catechin, quercetin, anthocyanin and chlorogenic acid, saponins such as soybean saponin, quillaia saponin and carrot saponin, polysaccharides such as chitinchitosan, fucoidan, β-glucan, mannan and water-soluble soybean polysaccharide, vitamins such as α-carotene, β-carotene, lutein, α-tocopherol and L-ascorbic acid, peptides such as soybean peptide, milk peptide and collagen peptide, carotenoids such as lycopene, sesame lignan, astaxanthin and β-cryptoxanthin, zinc, limonene, momordicine, charantin, sesaminol, laetrile, isothiocyanate, curcumin, propolis, coenzyme Q10, and soybean protein can be used as the anti-cancer ingredient and/or cancer preventive ingredient.

When the composition is a medicine, in addition to the above-described ingredients, pharmaceutically acceptable ingredients can be used without specific limitation.

Although a conventional carcinostatic agent acts to kill cancer cells by directly affecting the cancer cells in a cancer patient, it is difficult to eradicate cancer cells by a conventional carcinostatic agent. If any one of the cancer cells having cancer metastatic activity remains, there is the possibility of cancer metastasis to other organs. Therefore, a conventional carcinostatic agent can not inhibit cancer metastasis. In addition, a conventional carcinostatic agent may affect not only cancer cells but also normal cells. The composition of the present invention does not have such a cell-killing effect and can inhibit the cancer metastatic activity of cancer cells. Therefore, use of the composition of the present invention can reduce a burden imposed on a human body which is caused by excessive or long-term administration of a carcinostatic agent, and may fundamentally solve problems of treatment and prevention of cancer.

Examples are described bellow, but the technological idea of the present invention should not be limited by the Examples.

### Examples

### <Preparation Example 1> Preparation of soybean whey

To low-denatured defatted soybeans was added 10-time larger amount of warm water, and the mixture was extracted under stirring for 1 hour while keeping pH 7.0. Okara (soybean curd refuse), which is an insoluble component, was removed by decanter to obtain defatted soymilk. The defatted soymilk was adjusted to pH 4.5 by addition of concentrated hydrochloric acid, and precipitated soybean protein was removed to obtain soybean whey.

### <Preparation Example 2> Preparation of KTI and BBI

200 kg of the soybean whey obtained in Preparation Example 1 (dry solid content: 3.0% by weight; pH 4.6; total trypsin inhibitor potency: 960,000 units; crude protein content: 20.3 dry%; ash content: 20.1 dry%; carbohydrate content: 59.6 dry%) was concentrated under reduced pressure at 50°C of evaporating temperature and at 80°C of heating temperature of soybean whey with EVAPOR (CEP-L model, manufactured by Okawara Mfg. Co., Ltd.), to obtain 14,000 g of concentrated whey (dry solid content: 42.2% by weight of; total trypsin inhibitor potency: 930,000 units (66 unit/g)). (There was about 200g of loss due to adhesion and the like).

The concentrated whey was cooled to 15°C, adjusted to pH 4.0 with phosphoric acid, allowed to stand at 10°C for 3 hours, and then centrifuged (1,500 G x 10 minutes) to separate it into a supernatant and precipitates. 12,850 g of the separated supernatant was obtained, and had a dry solid content of 41.3% by weight and a total trypsin inhibitor potency of 110,500 units (8.6 units/g). 1,150 g of the precipitates were obtained, and had a dry solid content of 52.3% by weight and a total trypsin inhibitor potency of 820,000 units (713 units/g). Accordingly, 11.9% of trypsin inhibitors were present in the supernatant, and 88.1% of trypsin inhibitors were present in the precipitates.

Then, to 1,000 g of the precipitate was added 2,000 g of water. The mixture was stirred with a homomixer (4,000 rpm x 15 minutes) while keeping pH 4.0 to dissolve the precipitates, and then centrifuged (5,000 G x 10 minutes) to separate it into a supernatant (BBI) and precipitates (KTI). 2,767 g of the supernatant (BBI) was obtained, and had a solid content of 14.2% and a total trypsin inhibitor potency of 595,000 units (215 units/g). 233 g of the precipitate (KTI) was obtained, and had a solid content of 55.8% and a total trypsin inhibitor potency of 221,000 units (948 units/g). Each of them was freeze-dried, and used in the next experiments.

### <Test Example>

### Preparation of reagents

The KTI and BBI obtained in Preparation Example 2 were used in the following experiments.

Urokinase, synthetic substrate, plasminogen and plasmin were purchased from American Diagnostica (Greenwich, CT), and anti-ERK, anti-MEK1/2, anti-Akt, anti-phosphorylated ERK, anti-phosphorylated MEK1/2 and anti-phosphorylated Akt antibodies were purchased from New England Biolabs (Beverly, MA).

### Cultured cancer cell

Cultured mouse lung cancer cell 3LL (Chugai Pharmaceutical Co., Ltd.) was used. C57BL/6 black mice were used in experiments. In the experiments, human ovarian cancer cell HRA was transplanted into nude mice.

### Preparation method of culture medium

1 x 10⁶ cancer cells were placed in a 12-well petri dish and cultured for 16 hours. Then, the cancer cells attached to the wall of the petri dish were washed, and 0.3 ml of fresh culture medium was added into the petri dish. At this time, KTI or BBI was added together with the culture medium so that the concentration thereof becomes 0.1, 1.0 or 10 µM. In addition, G-CSF (granulocyte-colony stimulating factor) was added to a culture medium to stimulate urokinase production in cancer cells and in the system thus obtained that strongly expresses urokinase, the similar experiment was conducted. After 24 hours, the culture media were collected. Protein was measured by using a quantitative measurement kit for protein manufactured by Bio-Rad.

### <Evaluation of urokinase production inhibitory effects of KTI and BBI in mouse lung cancer cell 3LL>

To each of the culture media was added a sample buffer and the mixture was subjected to electrophoresis. On the resultant gel, a casein gel was placed, and bands dissolved by urokinase were quantified with a densitometer.

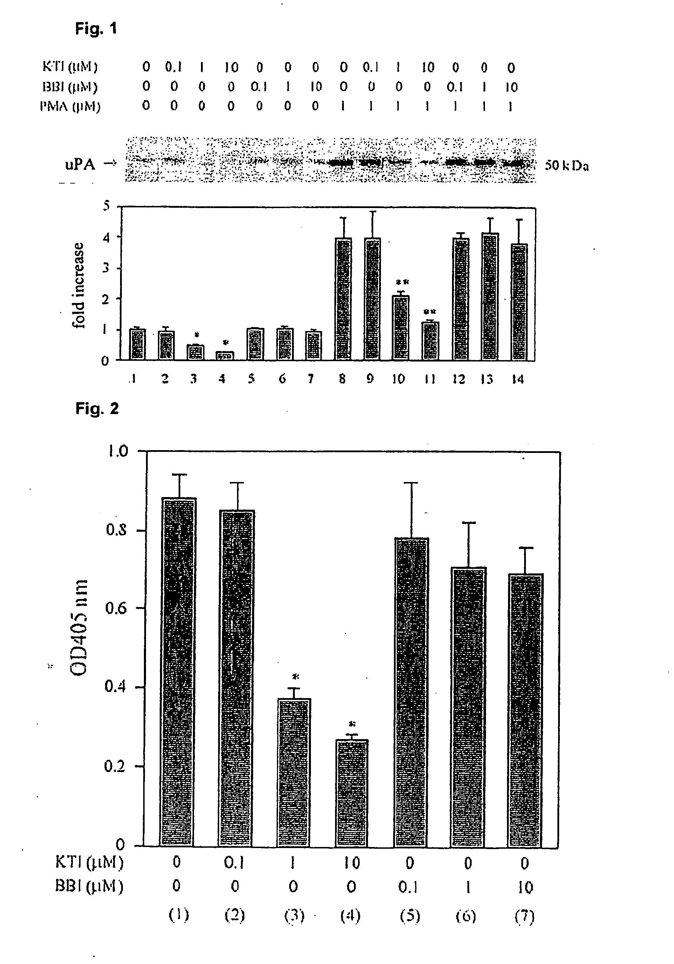

The results are shown in Fig. 1. The larger dissolution area of a band shows a larger amount of active urokinase expressed. When KTI or BBI was added for 12 hours in a concentration of 0.1, 1.0 or 10 µM, the addition of KTI (band 2 to 4) inhibited expression of urokinase production in the cancer cells, depending on the addition amount, whereas the addition of BBI (band 5 to 7) did not show the urokinase expression inhibitory effect. In the case of cancer cells stimulated with G-CSF (0.1 µg/ml) to enhance urokinase production, similarly as above, the addition of KTI (band 9 to 11) inhibited expression of urokinase production depending on the addition amount, whereas the addition of BBI (band 12 to 14) did not inhibit expression of urokinase production.

### <Evaluation of urokinase activity expressed on the cancer cell surface>

1 x 10⁴ cancer cells were placed in a 96-well petri dish and cultured for 16 hours. Then, the cancer cells attached to the wall of the petri dish were washed, and 0.1 ml of fresh culture medium was added into the petri dish. At this time, KTI or BBI was added together with the culture medium so that the concentration thereof becomes 0.1, 1.0 or 10 µM. In addition, the similar experiment was conducted in a system to which G-CSF was added to stimulate urokinase production in cancer cells. After 24 hours, the culture media were discarded. To the petri dish was added a synthetic substrate for urokinase measurement, and the yellow color of paranitroaniline was measured at OD 405 nm.

The results are shown in Fig. 2. Urokinase activity on the cancer cell surface was inhibited by KTI depending on the addition amount thereof (band 2 to 4). However, the addition of BBI did not show inhibition of urokinase activity (band 5 to 7).

### <Cancer invasion inhibitory effects of KTI and BBI in mouse lung cancer cell 3LL>

### Measurement of invasion ability of cancer cell

For an assay of cancer cell invasion using a Boyden chamber, 10⁵ cancer cells were placed in the upper chamber. After 24 hours, the number of cells that migrated to the bottom surface of a filter was counted. Using KTI or BBI in a concentration of 0.1, 1.0 or 10 µM, the degree of cancer invasion was evaluated by the Boyden chamber assay. For evaluating the invasion ability, the filter was coated with Matrigel, which is an artificial basement membrane.

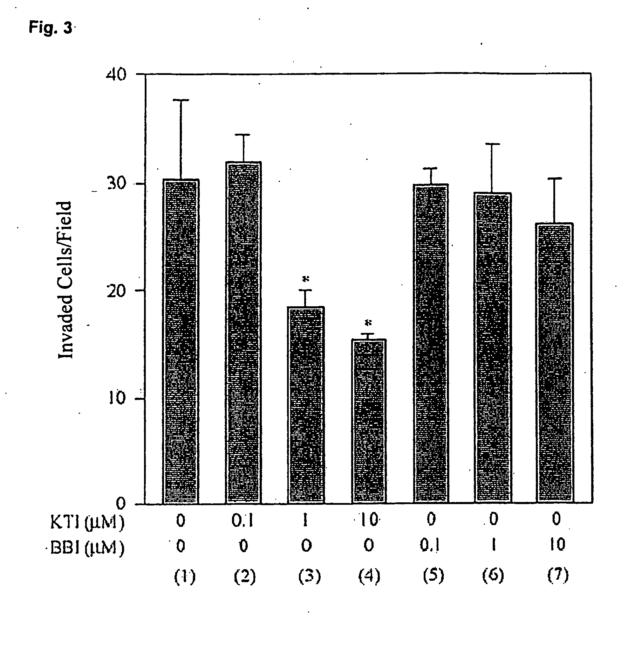

The results are shown in Fig. 3. Cancer invasion was inhibited by KTI depending on the addition amount thereof (band 2 to 4). However, BBI did not show the cancer invasion inhibitory effect (band 5 to 7).

### <Lung metastasis inhibitory effects of KTI and BBI in mouse lung cancer cell 3LL (Experimental metastasis)>

The experimental metastasis method is a method of compulsorily injecting a cancer cell into a blood vessel by intravenously injecting the cancer cell to a mouse via a tail vain at 2.5 x 10⁵ cells/50 µl/mouse. Mice were given 1 kg of standard feed mixed with 5, 15 or 50 g of KTI or BBI everyday and after for three weeks, the numbers of lung metastatic colonies were compared.

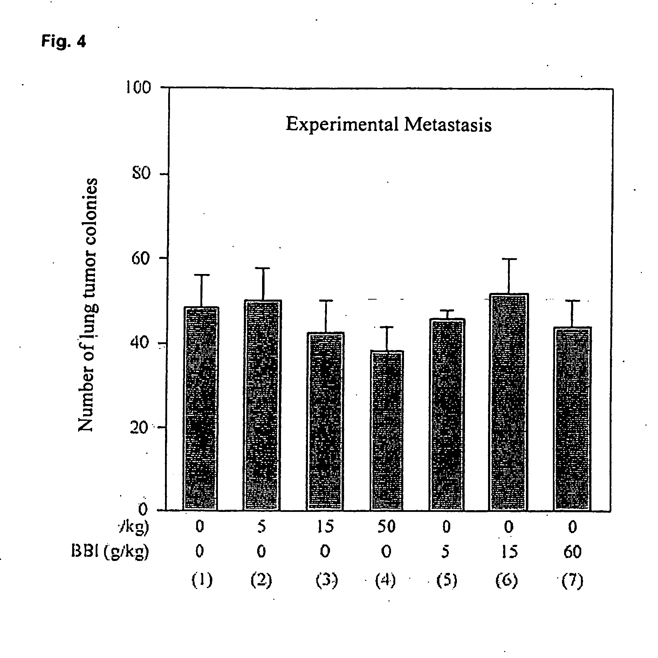

The result is shown in Fig. 4. Both of KTI (band 2 to 4) and BBI (band 5 to 7) did not lung metastases.

### <Lung metastasis inhibitory effects of KTI and BBI in mouse lung cancer cell 3LL (Spontaneous metastasis)>

The spontaneous metastasis method is a method of observing lung metastases after abdominal subcutaneous transplantation of a cancer cell into a mouse at 1.0 x 10⁶ cells/50 µl/mouse. Mice were given 1 kg of standard feed mixed with 5, 15 or 50 g of KTI or BBI everyday and after four weeks, the numbers of lung metastatic colonies were compared.

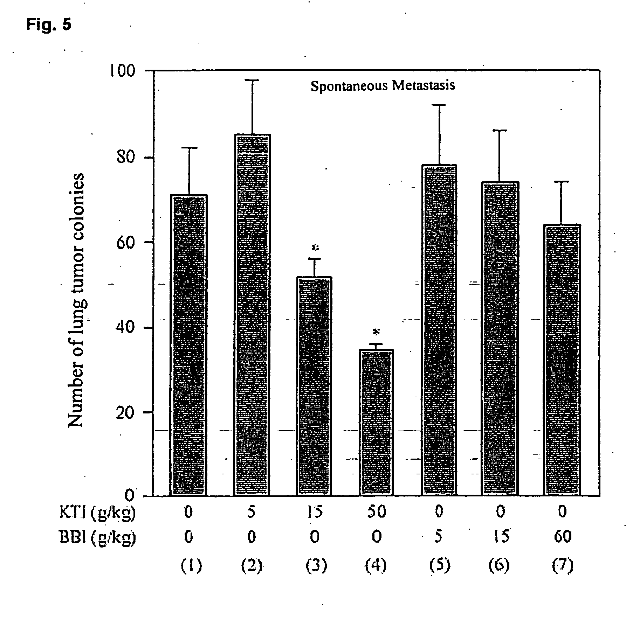

The result is shown in Fig. 5. Lung metastasis was inhibited only by KTI depending on the addition amount thereof (band 2 to 4). However, BBI did not show the lung metastasis inhibitory effect (band 5 to 7).

When a cancer cell was transplanted to a mouse subcutaneously, the cancer cell firstly proliferates at the transplanted site, induce angiogenesis to invade the blood vessel of the mouse, and then migrates from the blood vessel to an objective lung capillary to adhere thereto, so that it form lung metastasis. Since KTI had no inhibitory effect on Experimental metastasis but inhibited Spontaneous metastasis, KTI was found to inhibit some stage in the process of invasion of a cancer cell into a blood vessel. It is thought that once a cancer cell invades a blood vessel, the cancer metastasis inhibitory effect of KTI is reduced.

### <Peritonitis carcinomatosa inhibitory effects of KTI and BBI in human ovarian cancer cell HRA (Peritoneal disseminated metastasis)>

The peritoneal disseminated metastasis method is a method of causing peritonitis carcinomatosa by administering a cancer cell to a nude mouse intraperitoneally at 5 x 10⁶ cells/mouse to compulsorily inject the cancer cell intraperitoneally. Mice were given 1 kg of standard feed mixed with 5, 15 or 50 g of KTI or BBI everyday, and on the ninth day, the degrees of peritonitis.carcinomatosa were compared.

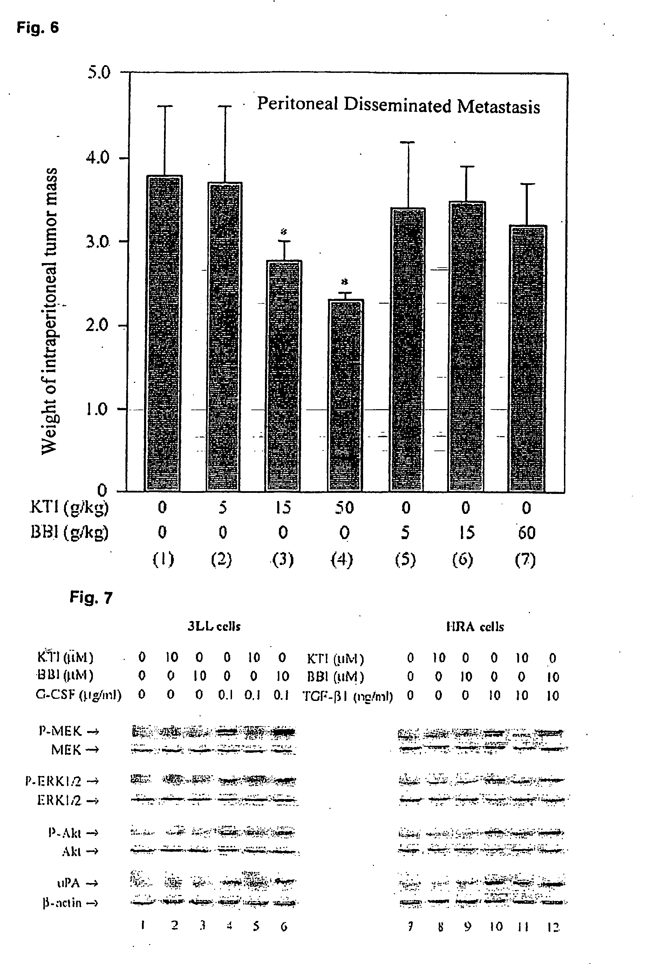

The result is shown in Fig. 6. Peritonitis carcinomatosa was inhibited only by KTI depending on the addition amount thereof (band 2 to 4). However, BBI did not show the peritonitis carcinomatosa inhibitory effect (band 5 to 7).

### <Signal transduction inhibitory effects of KTI and BBI in mouse lung cancer cell 3LL and human ovarian cancer cell HRA>

### Western blotting

A protein was subjected to electrophoresis, then transferred to a nitrocellulose membrane, and then analyzed by Western blotting with various antibodies (0.1µg/ml).

A cancer cell uses various growth factors and cytokines for proliferation and metastasis. As for cancer metastasis of interest, destruction of an extracellular matrix by urokinase is the most important. In the cancer cell 3LL used in the present invention, it has been known that its urokinase production is increased by G-CSF. In HRA cell, it has been confirmed that its urokinase production is increased by stimulation with TGF-β1 (transforming growth factor-beta 1). The increased urokinase production is associated with the signal transduction that binding of G-CSF or TGF-β1 to a receptor on the membrane of a cancer cell activates (phosphorylates) a MAP kinase (MEK, ERK) or PI3 kinase (Akt) and finally results in enhanced urokinase expression.

As shown in Fig. 7, it was found that, when KTI or BBI was added in combination with G-CSF or TGF-β1, the addition of KTI inhibited phosphorylation of MEK, ERK and Akt (band 2, 5, 8, 11), resulting in inhibition of urokinase expression. BBI did not show an inhibiting effect on the signal transduction (band 3, 6, 9, 12). The result suggests that KTI inhibits cancer metastasis by braking the signal transduction of urokinase production.

As described above, the KTI derived from soybean was found to inhibit the process of the invasion of mouse lung cancer cells from a primary lesion into a blood vessel. In the process, it is presumed that KTI inhibits urokinase from destroying connective tissues surrounding cancer cells to result in the infiltrative metastases of the cancer cells. Once a cancer cell invaded into a blood vessel, it was difficult to inhibit the metastasis of the cancer cell. Thus, KTI was found to inhibit the early invasion process of cancer cells.

It is thought that cancer cells destroy enzymatically connective tissues surrounding the cancer cells to result in the cancer invasion. For the enzyme, urokinase and matrix metalloproteinase (MMP) are important. It is thought that KTI inhibits cancer metastasis by inhibiting the urokinase production from cancer cells.

### Industrial Applicability

In the medical field, specifically, if the number of deaths from cancer in Japan is 170,000 men and 110,000 women, the number of patients to whom a cancer metastasis inhibitor will be administered is expected to be about 300,000 at lowest. To coexist with cancer, the patient must take a cancer metastasis inhibitor for a long time. However, a cancer metastasis inhibitor is essentially intended for use in relatively early cancer. It is though that, when trying to radically inhibit cancer metastasis, a patient ideally continues taking a cancer metastasis inhibitor for several years during which the patient is at risk of cancer recurrence. Considering those above described together, 500,000 or more patients are considered to be the subjects to whom the medicine and/or food product of the present invention is used.

There is an increasing demand for studies on inhibition of cancer metastasis in pharmaceutical companies, as well as universities and research institutes. Further, the bioinformatics market has broadened to the medical field, the food industry and the environment-related business. Some bioinformatics companies run their business in the environmental field, and some intend to develop their business in the food industry or the environment-related business. It is expected that the bioinformatics market will increasingly expand in the future. Based on such a background, clinical application of the medicine of the present invention is expected to bring innovation in the pharmaceutical industry, and even to develop the industry of food products for health.

### Brief Description of Drawings

Fig. 1 is an electropherogram showing the urokinase production inhibitory effects of KTI and BBI in mouse lung cancer cell 3LL and a graph showing values measured by a densitometer.
Fig. 2 is a graph showing urokinase activities on the surface of mouse lung cancer cell 3LL with KTI and BBI.
Fig. 3 is a graph showing the cancer invasion inhibitory effects of KTI and BBI in mouse lung cancer cell 3LL measured by the Boyden chamber assay.
Fig. 4 is a graph showing the lung metastasis inhibitory effects of KTI and BBI in mouse lung cancer cell 3LL measured by the Experimental metastasis method.
Fig. 5 is a graph showing the lung metastasis inhibitory effects of KTI and BBI in mouse lung cancer cell 3LL measured by the Spontaneous metastasis method.
Fig. 6 is a graph showing the carcinomatous peritonitis inhibitory effects of KTI and BBI in mouse lung cancer cell 3LL measured by the Peritoneal disseminated metastasis method.
Fig. 7 is a graph showing the signal transduction inhibitory effects of KTI and BBI in mouse lung cancer cell 3LL and human ovarian cancer cell HRA.

## Claims

1. A composition comprising a soybean Kunitz trypsin inhibitor as an active ingredient for use in inhibiting cancer metastasis.

2. The composition according to claim 1, which comprises soybean whey as a source of the soybean Kunitz trypsin inhibitor.

3. The composition according to claim 1, which comprises a trypsin inhibitor concentrate obtained from soybean whey as a source of the soybean Kunitz trypsin inhibitor.

4. The composition according to claim 3, wherein a Bowman-Birk trypsin inhibitor is separated and removed as much as possible from the trypsin inhibitor concentrate obtained from soybean whey.

5. The composition according to claim 1, which is a food product or medicine.

6. The composition according to claim 1 further comprising an anti-cancer ingredient and/or a cancer preventive ingredient.

## Patentansprüche

1. Zusammensetzung, die einen Soja-Kunitz-Trypsin-Inhibitor als Wirkstoff zur Verwendung bei der Hemmung der Krebsmetastasierung umfasst.

2. Zusammensetzung gemäß Anspruch 1, die Sojamolke als Quelle für den Soja-Kunitz-Trypsin-Inhibitor umfasst.

3. Zusammensetzung gemäß Anspruch 1, die ein aus Sojamolke erhaltenes Trypsin-Inhibitor-Konzentrat als Quelle für den Soja-Kunitz-Trypsin-Inhibitor umfasst.

4. Zusammensetzung gemäß Anspruch 3, wobei ein Bowman-Birk-Trypsin-Inhibitor soweit wie möglich aus dem aus Sojamolke erhaltenen Trypsin-Inhibitor-Konzentrat abgetrennt und entfernt wird.

5. Zusammensetzung gemäß Anspruch 1, bei der es sich um ein Lebensmittel oder Arzneimittel handelt.

6. Zusammensetzung gemäß Anspruch 1, die weiterhin einen Antikrebswirkstoff und/oder einen Krebspräventionswirkstoff umfasst.

## Revendications

1. Composition comprenant un inhibiteur trypsique de Kunitz du soja à titre de principe actif, destinée à être utilisée pour inhiber la métastase cancéreuse.

2. Composition selon la revendication 1, qui comprend du petit-lait de soja à titre de source d'inhibiteur trypsique de Kunitz du soja.

3. Composition selon la revendication 1, qui comprend un concentré d'inhibiteur trypsique obtenu à partir du petit-lait du soja à titre de source d'inhibiteur trypsique de Kunitz du soja.

4. Composition selon la revendication 3, dans laquelle un inhibiteur trypsique de Bowman-Birk est séparé et éliminé autant que possible du concentré d'inhibiteur trypsique obtenu à partir du petit-lait de soja.

5. Composition selon la revendication 1, qui est un produit alimentaire ou un médicament.

6. Composition selon la revendication 1 comprenant, en outre, un anticancéreux et/ou un agent de prévention contre le cancer.
